# DEMANDE DE BREVET EUROPEEN

(11) **EP 0 796 635 A1**
(43) Date de publication de la demande: **24.09.1997**
(21) Numéro de dépôt: 97400563.9
(22) Date de dépôt: 13.03.1997
(51) Int. Cl.: A61N 1/32

(54) **Appareil de stimulation électrique indolore**

(30) Priorité: 20.03.1996 FR 9603462
(71) Demandeur: SEB S.A., 69130 Ecully (FR)
(72) Inventeur: Bontoux, Daniel, 69230 Saint-Genis-Laval (FR); Paget, Monique, 69008 Lyon (FR); Debourg, Jean-Pierre, 69008 Lyon (FR)
(74) Mandataire: Michelet, Alain

(57) **Abrégé**

L'invention concerne un appareil de stimulation électrique indolore pour soins corporels.

Il comprend au moins une électrode de stimulation (24, 25) appliquée localement sur la peau, ainsi qu'une pièce vibrante (23) disposée au voisinage de l'électrode, qui sollicite mécaniquement la peau en vibration en combinaison avec la stimulation électrique. La pièce vibrante est actionnée mécaniquement par la rotation autour d'un axe (18) d'un organe rotatif (22), lui-même entraîné mécaniquement en rotation.

## Description

La présente invention concerne un appareil de stimulation électrique pour soins corporels et un procédé de stimulation électrique correspondant. Elle se rapporte ainsi aux appareils qui provoquent une stimulation par courants électriques appliqués par des électrodes au contact de la peau, relatifs à la beauté et au soin du corps.

On connaît de nombreux appareils utilisant la stimulation électrique.

On connaît par exemple des appareils dits antirides munis d'une électrode de petit diamètre qu'on applique sur les rides en la déplaçant, une deuxième électrode étant tenue à la main en même temps que le boîtier de l'appareil. Des courants non dangereux sont appliqués et provoquent une contraction locale bien ciblée des muscles peauciers. Cet exercice répété permet l'atténuation des rides. Mais bien que les courants appliqués de façon intermittente ne soient pas dangereux, ils sont ressentis par de nombreux sujets comme très désagréables. Ceci conduit à en baisser l'intensité et par conséquent à en diminuer l'efficacité de façon considérable.

On connaît également des appareils de tonification du visage comportant deux électrodes entre lesquelles sont appliquées des tensions intermittentes. La contraction et le relâchement répétés des muscles du visage les fortifient, ce qui a une action tonifiante. Mais les inconvénients décrits plus haut se retrouvent ici de la même façon.

Il a aussi été proposé des appareils de gymnastique passive comportant des électrodes que l'on dispose sur le corps. Ces électrodes permettent l'application sur la surface de la peau de courants qui font travailler par exemple les muscles ventraux, avec une action raffermissante, l'exercice permettant aussi de consommer les graisses.

Malheureusement, tous ces appareils pour être efficaces doivent faire passer en surface de la peau des courants qui, pour être non dangereux, n'en sont pas moins douloureux ou très désagréables.

Le document EP-A-0.544.544 concerne un dispositif de soulagement de douleur. Ce dispositif inclut une pluralité de sources de soulagement de douleur sélectionnées parmi un massage, une vibration, de la pression et une stimulation électrique, ainsi que des moyens d'application d'au moins deux de ces sources simultanément en un point sélectionné de la partie supérieure du corps d'une personne.

Le document FR-A-2.706.131 porte sur un casque stimulant la repousse des cheveux. Le casque est équipé intérieurement d'électrodes coulissantes s'adaptant au crâne et recevant d'un module électronique des impulsions électriques réglables. Le casque comporte aussi un dispositif vibrant mécanique incorporé.

Le document US-A-4.010.742 décrit un dispositif de thérapie électronique pour le traitement de divers maux du corps humain. Ce dispositif a un vibrateur incluant une source de chaleur et un circuit électrique qui fournit des électrons à la peau du corps humain au moyen de rouleaux mis en contact avec la peau.

Le document WO-A-95.04.516 concerne une méthode de massage du visage et du cou avec un dispositif appliquant une stimulation électrique suivie d'un massage manuel et d'un traitement vibratoire.

Le document FR-A-2.563.437 décrit un appareil pour favoriser la pénétration et l'imprégnation en profondeur, en particulier de la peau, par des produits cosmétiques. L'appareil présente deux sorties conformées pour autoriser chacune le branchement électrique d'un organe adaptateur. Au moins l'un des organes est agencé à son extrémité pour l'adaptation d'un accessoire, de manière à autoriser la transmission d'une vibration et la conduction d'un courant.

La présente invention a pour but un appareil de stimulation électrique pour soins corporels, supprimant ou diminuant sensiblement les effets douloureux des courants électriques appliqués.

L'invention a également pour but un tel appareil de stimulation électrique pouvant être compact et facile à utiliser.

L'invention vise également un tel appareil de stimulation électrique pouvant être de réalisation économique.

La présente invention a également pour but un procédé de stimulation électrique pour soins corporels, effaçant ou réduisant sensiblement la douleur et les effets désagréables.

L'invention a ainsi pour objet un appareil de stimulation électrique pour soins corporels, comprenant au moins une électrode de stimulation appliquée localement sur la peau et lui envoyant des courants lors d'une utilisation.

Cet appareil de stimulation électrique comprend au moins une pièce vibrante sollicitant mécaniquement la peau en vibration en combinaison avec la stimulation électrique, et un boîtier tenu à la main lors d'une utilisation.

Selon l'invention, la pièce vibrante étant disposée au voisinage des électrodes est actionnée mécaniquement par la rotation autour d'un axe d'au moins un organe rotatif, cet organe rotatif étant entraîné mécaniquement en rotation.

Une interprétation de ce phénomène d'effacement ou de réduction de la couleur est que la sollicitation mécanique en vibration de l'appareil de stimulation électrique sature les terminaisons nerveuses voisines des électrodes par des informations entretenues de façon permanente, ce qui masque les effets douloureux du courant électrique. En effet, quand on superpose à une sollicitation douloureuse, notamment intermittente, une sollicitation vibratoire et continue bien ajustée, l'influx nerveux est saturé par les vibrations et on ne ressent plus la douleur.

Il est important que les électrodes et la, ou les, pièces vibrantes coopèrent en vue d'une stimulation électrique indolore. Cela est obtenu lorsque l'appareil de stimulation électrique assure une sollicitation mécanique au voisinage des électrodes.

Soit les vibrations mécaniques sont appliquées à proximité des électrodes, l'appareil de stimulation électrique remplissant une fonction de vibreur purement mécanique, soit elles soit obtenues en faisant vibrer les électrodes elles mêmes, l'appareil de stimulation électrique remplissant alors une fonction de vibreur électromécanique.

Avantageusement, la pièce vibrante produit en fonctionnement une vibration mécanique à une fréquence de vibration approximativement égale à 100 Hz.

En effet, l'atténuation de la douleur est la plus efficace avec une sollicitation vibratoire dont la fréquence est de cet ordre. Il est en tout cas souhaitable que la fréquence de vibration ne dépasse pas 300 Hz, la réponse nerveuse n'étant plus proportionnelle à la fréquence de sollicitation au-delà.

De préférence, la pièce vibrante produit en fonctionnement une vibration mécanique ayant une amplitude comprise entre 1/5 de mm et 1 mm.

Dans une forme avantageuse de réalisation, l'appareil de stimulation électrique comprend une partie métallique externe au boîtier par laquelle le boîtier est tenu à la main, cette partie métallique faisant office d'électrode de masse.

Dans un premier mode de réalisation préféré, la pièce vibrante est dissociée des électrodes.

Dans un second mode de réalisation préféré de l'appareil de stimulation électrique selon l'invention, la pièce vibrante porte au moins une des électrodes de stimulation.

Selon une première forme avantageuse de ce second mode de réalisation, les électrodes de stimulation étant deux électrodes approximativement parallèles appliquées simultanément sur une surface de la peau en fonctionnement, l'appareil comprend:
- un boîtier,
- un support portant les deux électrodes de stimulation,
- des moyens élastiques reliant de manière souple le support au boîtier,
- une masselotte disposée sur le support selon un axe de rotation le traversant, la masselotte étant excentrée par rapport à l'axe,
- et des moyens d'entraînement de la masselotte en rotation,
de telle sorte qu'un entraînement en rotation de la masselotte provoque un mouvement du support porteur des électrodes de stimulation approximativement perpendiculaire à la surface de la peau.

Dans une seconde forme avantageuse de ce second mode de réalisation, l'appareil de stimulation électrique comprend:
- un boiter,
- deux rouleaux ayant des axes approximativement parallèles autour desquels ils sont mobiles, les rouleaux étant reliés au boîtier et alimentés à une source de tension de façon à constituer les électrodes de stimulation appliquées sur la peau en fonctionnement,
- des moyens d'entraînement d'au moins un des rouleaux en rotation vibratoire, sollicitant mécaniquement la peau en vibration.

Dans cette seconde forme avantageuse de réalisation, il est intéressant que l'appareil de stimulation comprenne également des moyens d'entraînement du rouleau en rotation continue, les moyens d'entraînement en rotation continue et en rotation vibratoire ayant des effets superposés.

De plus, il est avantageux qu'un premier des rouleaux étant entraîné par les moyens d'entraînement, le second rouleau soit freiné en rotation, de façon à faciliter la formation d'un bourrelet sur la peau lors d'une application de l'appareil.

L'invention a également pour objet un procédé de stimulation électrique pour soins corporels de nature esthétique ou fortifiante.

Selon l'invention, on applique simultanément et dans un même voisinage local de la peau la stimulation électrique et une sollicitation mécanique vibratoire produite par l'actionnement mécanique d'au moins une pièce vibrante, au moyen de la rotation autour d'un axe d'au moins un organe rotatif entraîné mécaniquement en rotation.

D'autres particularités et avantages de l'appareil et du procédé selon l'invention ressortiront de la description suivante, donnée en référence aux dessins annexés à simple titre indicatif, et sur lesquels:
- La Figure 1 montre selon une section transversale, un premier mode de réalisation de l'appareil de stimulation électrique selon l'invention.
- La Figure 2 schématise en coupe longitudinale un second mode de réalisation de l'appareil I de stimulation électrique selon l'invention.

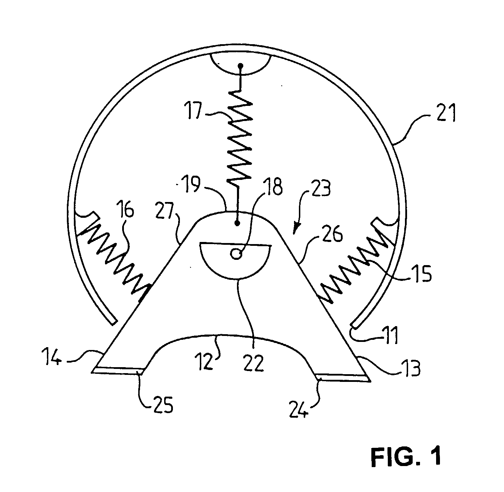

Dans un premier mode de réalisation de l'appareil de stimulation électrique selon l'invention, représenté sur la Figure 1, celui-ci comprend deux électrodes de stimulation 24 et 25 appliquées sur la peau, produisant elles-mêmes des vibrations.

L'appareil de stimulation électrique dans ce premier mode de réalisation comprend un corps 21 ou boîtier, approximativement cylindrique circulaire, et une pièce vibrante 23. Cette dernière a une forme massive de section approximativement triangulaire. La section de la pièce vibrante 23 comporte une base 12, un sommet 19 et des parois latérales 26 et 27 reliant la base 12 au sommet 19. Des pattes 13 et 14 débordent latéralement de la base 12 dans la prolongation des parois latérales 26 et 27. Les électrodes de stimulation 24 et 25 sont placées respectivement à leurs extrémités. Ainsi, les électrodes de stimulation 24 et 25 sont larges et écartées.

A titre d'exemple, la largeur des électrodes de stimulation 24 et 25 vaut 8 mm et leur écartement 20 à 30 mm.

La plus grande partie de la pièce vibrante 23 est disposée dans le corps 21, le sommet 19 étant orienté vers l'intérieur du corps 21, et la base 12 sortant du corps 21 par une ouverture 11.

La pièce vibrante 23 est rattachée au corps 21 par des moyens élastiques permettant une liaison souple. Ceux-ci consistent par exemple en des ressorts 15, 16 et 17 respectivement reliés aux parois latérales 26 et 27 et au sommet 19.

De plus, la pièce vibrante 23 porte un arbre 18 disposé à environ un tiers du chemin entre le sommet 19 et la base 12. Cet arbre 18 est mobile de manière souple à l'intérieur du corps 21, étant par exemple rattaché à un arbre de position fixe par un joint élastique. L'arbre 18 est solidaire d'une masselotte 22 excentrée, ayant approximativement une forme de demi-disque, disposée sur la pièce vibrante 23. Le corps 21 de l'appareil contient un moteur électrique entraînant en fonctionnement l'arbre 18 en rotation, et provoquant ainsi un mouvement de la pièce vibrante 23 à travers l'ouverture 11, approximativement de translation.

Lors d'une utilisation, les électrodes de stimulation 24 et 25 sont alimentées électriquement, par exemple par une électronique interne, et le moteur électrique fait tourner la masselotte 22 qui met en mouvement la pièce vibrante 23. Les électrodes de stimulation 24 et 25 en mouvement sont appliquées périodiquement sur la peau, et lui communiquent ainsi des vibrations avant des impulsions électriques. Leur largeur et leur écartement permet aux impulsions électriques émises de provoquer une contraction par impulsions d'une zone assez grande de la peau, située entre elles et à leur voisinage. De ce fait, elles ont une action augmentant la musculation, et par conséquent tonifiante. Les vibrations de la pièce vibrante 23 sont telles que les impulsions électriques ne sont pas ou sont peu ressenties.

Avantageusement, l'arbre 18 est mis en rotation à une fréquence de l'ordre de 100 Hz.

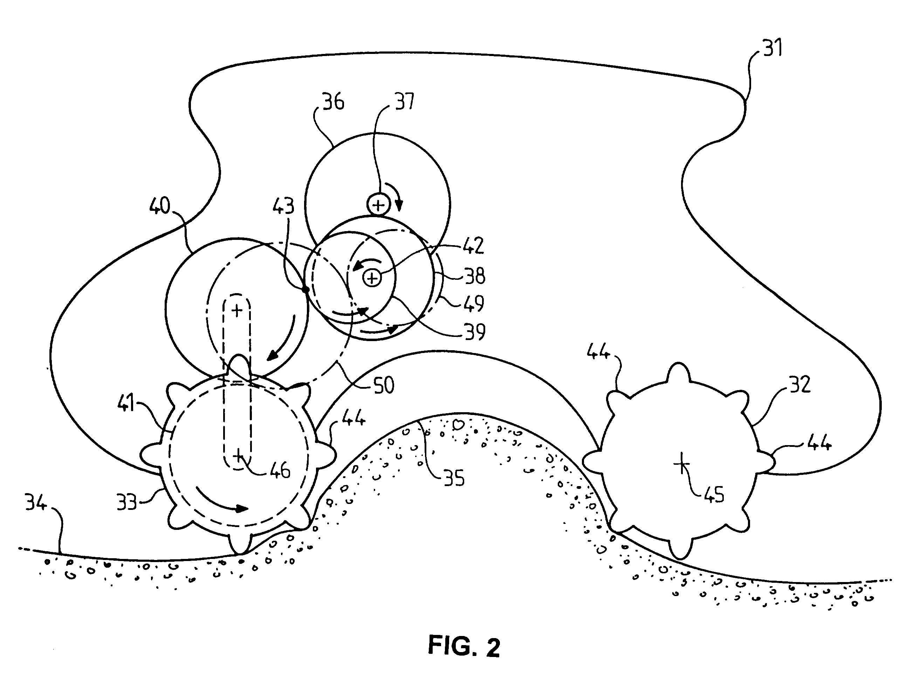

Dans un second mode de réalisation, schématisé sur la Figure 2, l'appareil de stimulation électrique comprend un corps 31 ou boîtier pouvant être tenu à la main, et deux rouleaux 32 et 33 partiellement contenus dans le corps 31. Les rouleaux 32 et 33 ont des axes respectivement 45 et 46 parallèles, et sont munis de dents 44. A titre d'exemple, chacun des rouleaux 32, 33 comporte huit dents 44. Un premier des rouleaux 32 est freiné, tandis que le second 33 est mis en rotation par des moyens d'entraînement. Les rouleaux 32 et 33 sont conducteurs d'électricité et alimentés à une source de tension, de façon à constituer les électrodes de stimulation appliquées sur la peau. Par exemple, ils sont recouverts par un dépôt d'argent ou sont composés d'un plastique conducteur.

Les moyens d'entraînement en rotation du second rouleau 33 comprennent un moteur 36 agissant sur le rouleau 33 par une chaîne cinématique. Cette chaîne cinématique provoque un mouvement de rotation qui comprend à la fois une composante continue et une composante alternative, cette dernière ayant une fréquence vibratoire. Dans l'exemple de réalisation, la chaîne cinématique comporte une première roue 37 engrené à une deuxième roue 38 fixée et normalement centrée sur un arbre 42. Une troisième roue 39 est fixée sur le même arbre 42, mais de façon excentrée. La troisième roue 39 est engrenée à une quatrième roue 40, elle-même engrenée à une cinquième roue 41 solidaire du second rouleau 33. La quatrième roue 40 oscille autour de la cinquième roue 41 de façon à maintenir l'engrenage avec la troisième roue 39.

Lors d'une utilisation, les rouleaux 32 et 33 sont appliqués simultanément sur la peau 34. Le moteur 36 est mis en route et entraîne le second rouleau 33 en rotation, tandis que le premier rouleau 32 est freiné. Il se forme ainsi un bourrelet de peau 35 entre les rouleaux 32 et 33, ce bourrelet 35 progressant en même temps que l'appareil se déplace. L'appareil se comporte ainsi comme un masseur à rouleaux parallèles effectuant un « palpé-roulé » de la peau 34. Simultanément, on applique entre les rouleaux 32 et 33 une tension intermittente de forme connue, et les courants générés dans le bourrelet 35 augmentent l'efficacité du massage. La contraction des muscles du bourrelet 35 consomme les graisses de façon bien plus efficaces que sans stimulation électrique.

Grâce à la chaîne cinématique, un entraînement régulier et continu du moteur 36 provoque la superposition de mouvements de rotation continu et alternatif du second rouleau 33. Ainsi, dans la position indiquée sur la Figure 2 en traits continus, le point d'engrenage 43 des roues 39 et 40 est très écarté de l'arbre 42, de telle sorte que la quatrième roue 40 tourne vite, ainsi que le second rouleau 33. En revanche, dans une seconde position des roues 39 et 40, qui apparaissent respectivement en traits mixtes aux positions 49 et 50, le point d'engrenage 43 est proche de l'arbre 42 et la roue 40 tourne doucement, ainsi que le second rouleau 33. Ces variations de la vitesse de rotation du second rouleau 33 provoque les vibrations souhaitées

On règle facilement l'amplitude de la composante alternative par rapport à la composante continue en faisant varier l'excentration de la troisième roue 39, qui fait office de roue menante. Si l'arbre 42 est en dehors du cercle primitif de la troisième roue 39, on peut même avoir un rebroussement périodique du mouvement.

Les sollicitations vibratoires obtenues par l'appareil rendent la stimulation électrique efficace et indolore.

Préférentiellement, le mouvement de rotation alternatif a une fréquence de l'ordre de 100 Hz.

Les signes de référence insérés après les caractéristiques techniques mentionnées dans les revendications, ont pour seul but de faciliter la compréhension de ces dernières, et n'en limitent aucunement la portée.

## Revendications

1. Appareil de stimulation électrique pour soins corporels, comprenant au moins une électrode de stimulation (24, 25, 32, 33) appliquée localement sur la peau et lui envoyant des courants lors d'une utilisation, au moins une pièce vibrante (23, 33) sollicitant mécaniquement la peau en vibration en combinaison avec la stimulation électrique, et un boîtier (21, 31) tenue à la main lors d'une utilisation, caractérisé en ce que ladite pièce vibrante (23, 33) étant disposée au voisinage desdites électrodes (24, 25, 32, 33) est actionnée mécaniquement par la rotation autour d'un axe (18,46) d'au moins un organe rotatif (22, 33), ledit organe rotatif étant entraîné mécaniquement en rotation.

2. Appareil de stimulation électrique selon la revendication 1, caractérisé en ce que ladite pièce vibrante (23, 33) produit en fonctionnement une vibration mécanique à une fréquence de vibration approximativement égale à 100 Hz.

3. Appareil de stimulation électrique selon l'une des revendications 1 ou 2, caractérisé en ce que ladite pièce vibrante (23, 33) produit en fonctionnement une vibration mécanique ayant une amplitude comprise entre 1/5 de mm et I mm.

4. Appareil de stimulation électrique selon l'une quelconque des revendications 1 à 3, caractérisé en ce qu'il comprend une partie métallique externe au boîtier par laquelle le boîtier est tenu à la main, ladite partie métallique faisant office d'électrode de masse.

5. Appareil de stimulation électrique selon l'une quelconque des revendications 1 à 4, caractérisé en ce que la pièce vibrante est dissociée desdites électrodes.

6. Appareil de stimulation électrique selon l'une quelconque des revendications 1 à 4, caractérisé en ce que ladite pièce vibrante (23, 33) porte au moins une desdites électrodes de stimulation (24, 25, 32,33).

7. Appareil de stimulation électrique selon la revendication 6, caractérisé en ce que lesdites électrodes de stimulation (24, 25) étant deux électrodes approximativement parallèles appliquées simultanément sur une surface de la peau en fonctionnement, l'appareil comprend:
- un boîtier (21),
- un support (23) portant les deux électrodes de stimulation (24, 25),
- des moyens élastiques (15, 16, 17) reliant de manière souple le support (23) au boîtier (21),
- une masselotte (22) disposée sur le support (23) selon un axe de rotation (18) le traversant, la masselotte (22) étant excentrée par rapport audit axe (18),
- et des moyens d'entraînement de la masselotte (22) en rotation,
de telle sorte qu'un entraînement en rotation de la masselotte (22) provoque un mouvement du support (23) porteur des électrodes de stimulation (24, 25) approximativement perpendiculaire à la surface de la peau.

8. Appareil de stimulation électrique selon la revendication 6, caractérisé en ce qu'il comprend:
- un boiter (31),
- deux rouleaux (32, 33) ayant des axes (45, 46) approximativement parallèles autour desquels ils sont mobiles, lesdites rouleaux (32, 33) étant reliés au boîtier (31) et alimentés à une source de tension de façon à constituer les électrodes de stimulation appliquées sur la peau en fonctionnement,
- des moyens d'entraînement (36-42) d'au moins un des rouleaux (33) en rotation vibratoire, sollicitant mécaniquement la peau en vibration.

9. Appareil de stimulation selon la revendication 8, caractérisé en ce qu'il comprend également des moyens d'entraînement (36-42) dudit rouleau (33) en rotation continue, lesdits moyens d'entraînement en rotation continue et en rotation vibratoire ayant des effets superposés.

10. Appareil de stimulation selon l'une des revendications 8 ou 9, caractérisé en ce qu'un premier des rouleaux (33) étant entraîné par lesdits moyens d'entraînement (36-42), le second rouleau (32) est freiné en rotation, de façon à faciliter la formation d'un bourrelet (35) sur la peau (34) lors d'une application de l'appareil.

11. Procédé de stimulation électrique pour soins corporels de nature esthétique, caractérisé en ce qu'on applique simultanément et dans un même voisinage local de la peau (34), la stimulation électrique et une sollicitation mécanique vibratoire produite par l'actionnement mécanique d'au moins une pièce vibrante (23, 33), au moyen de la rotation autour d'un axe (18, 46) d'au moins un organe rotatif (22, 33) entraîné mécaniquement en rotation.

12. Procédé de stimulation électrique pour soins corporels de nature fortifiante, caractérisé en ce qu'on applique simultanément et dans un même voisinage local de la peau (34), la stimulation électrique et une sollicitation mécanique vibratoire produite par l'actionnement mécanique d'au moins une pièce vibrante (23, 33), au moyen de la rotation autour d'un axe (18, 46) d'au moins un organe rotatif (22, 33) entraîné mécaniquement en rotation.
